# FASCICULE DE BREVET EUROPEEN

(11) **EP 2 542 235 B1**
(45) Date de publication et mention de la délivrance du brevet: **22.11.2017**
(21) Numéro de dépôt: 11712951.0
(22) Date de dépôt: 02.03.2011
(51) Int. Cl.: A61K 31/282, A61K 31/428, A61K 45/06, A61K 49/00, A61P 25/00, A61P 35/00

(54) **UTILISATION DU RILUZOLE POUR TRAITER OU PRÉVENIR LES EFFETS INDÉSIRABLES D'AGENTS ANTI-CANCÉREUX**
VERWENDUNG VON RILUZOL ZUR BEHANDLUNG ODER VERMEIDUNG DER NEBENWIRKUNGEN VON ANTINEOPLASTIKA
USE OF RILUZOLE TO TREAT OR PREVENT THE ADVERSE EFFECTS OF ANTINEOPLASTIC AGENTS

(30) Priorité: 02.03.2010 FR 1051491
(43) Date de publication de la demande: 09.01.2013
(73) Titulaire: Universite d'Auvergne Clermont I, 63000 Clermont Ferrand (FR)
(72) Inventeur: BUSSEROLLES, Jérôme, 03800 Saulzet (FR); ALLOUI, Abdelkrim, 63000 Clermont Ferrand (FR); LAZDUNSKI, Michel, 06000 Nice (FR); ESCHALIER, Alain, 63400 Chamalières (FR)
(74) Mandataire: Lavoix
(86) Numéro de dépôt international: PCT/FR2011/050432
(87) Numéro de publication internationale: WO 2011/107710

(56) Documents cités:
- WO-A1-00/28992
- WO-A1-00/66121
- WO-A1-2006/099739
- WO-A1-2009/028605
- US-A- 4 536 386
- US-A- 5 624 945
- US-A1- 2002 151 465
- US-A1- 2006 188 445
- US-A1- 2008 124 333
- TODD RYAN C ET AL: "Inhibition of transcription by platinum antitumor compounds.", METALLOMICS : INTEGRATED BIOMETAL SCIENCE 2009 LNKD- PUBMED:20046924, vol. 1, no. 4, 2009, pages 280-291, XP002636711, ISSN: 1756-591X
- LEFEBVRE C ET AL: "Effects of pretreatment with anti-convulsant agents on the development of nerve injury-induced mechanical and cold hypersensitivity", ABSTRACTS OF THE ANNUAL MEETING OF THE SOCIETY FOR NEUROSCIENCE, SOCIETY FOR NEUROSCIENCE, WASHINGTON, DC, US, vol. 26, no. 1-2, 1 janvier 2000 (2000-01-01), page 1216, XP008136024, ISSN: 0190-5295
- HERNAANDEZ J ET AL: "THE MODULATION OF PROSTATE CANCER RISK WITH alpha-TOCOPHEROL: A PILOT RANDOMIZED, CONTROLLED CLINICAL TRIAL", JOURNAL OF UROLOGY, LIPPINCOTT WILLIAMS & WILKINS, BALTIMORE, MD, US LNKD- DOI:10.1097/01.JU.0000165151.08560.6A, vol. 174, no. 2, 1 août 2005 (2005-08-01), pages 519-522, XP025378144, ISSN: 0022-5347 [extrait le 2005-08-01]
- AUTHIER NICOLAS ET AL: "Assessment of allodynia and hyperalgesia after cisplatin administration to rats", NEUROSCIENCE LETTERS, vol. 291, no. 2, 15 septembre 2000 (2000-09-15), pages 73-76, XP002599724, ISSN: 0304-3940

## Description

La présente invention concerne le riluzole pour une utilisation pour traiter ou prévenir les effets indésirables liés à l'administration d'agents anticancéreux présentant des effets neurotoxiques, tels que par exemple les sels de platine, les taxanes et les alcaloïdes de la pervenche.

### Le riluzole

Le riluzole, qui est commercialisé par Aventis sous la dénomination commerciale de Rilutek®, est aujourd'hui indiqué pour prolonger la durée de vie ou retarder le recours à la ventilation mécanique assistée chez les patients atteints de sclérose latérale amyotrophique (Miller et al. Neurology 2009 73:1218-26).

### L'oxaliplatine

L'oxaliplatine est un agent anti-néoplasique, cytotoxique, composé d'un atome de platine complexé avec un 1,2-diaminohexane (DACH) et un groupe oxalate. Il est utilisé, entre autres, lors du traitement de cancers colorectaux. Il a récemment été employé dans le traitement du cancer colorectal en stade métastasique avancé (Baker, Rev Gastroenterol Disord. 2003 3:31-8 ; Screnci et al., Br J Cancer 2000 82:966-72) et a également montré une activité dans d'autres cancers : ovaire, sein, poumon (Muggia, Semin Oncol. 2004 31:17-24; Petit et al., Anticancer Drugs. 2006 17:337-43). La neurotoxicité est un effet indésirable fréquent de cette molécule et elle se présente souvent sous deux formes : aiguë et chronique.

La neurotoxicité aiguë est caractérisée par la survenue rapide d'une dysesthésie distale induite par le froid avec ou non paresthésies (Gamelin et al., Semin Oncol. 2002 29:21-33 ; Lehky et al., Muscle Nerve. 2004 29:387-92), et de troubles musculaires : myotonie, crampes, fasciculations musculaires prolongées affectant les jambes, les mains et les mâchoires et gênant le mouvement (Grolleau et al., J Neurophysiol. 2001 85:2293-7). Environ 85% à 95% des patients traités développent ces symptômes qui commencent pendant la perfusion et persistent pendant les 24 à 48 premières heures.

L'intensité de cette neurotoxicité aiguë dépend des concentrations plasmatiques d'oxaliplatine, et il convient donc éviter les pics de concentration (Grothey, Clin Colorectal Cancer. 2005 5:S38-S46). La résolution des symptômes se fait en environ une semaine, mais ils réapparaissent lors de la perfusion suivante. Comparé aux autres agents dérivés du platine, le profil neurotoxique de l'oxaliplatine est particulier en raison de cette neurotoxicité aiguë. Chez des patients présentant de tels symptômes aigus, les études ont montré peu ou pas de dégénérescence axonale, suggérant un effet spécifique de l'oxaliplatine sur les neurones sensitifs et les neurones moteurs. L'intensité des troubles observés et particulièrement de l'hypersensibilité au froid, après les premières cures, est un marqueur de neurotoxicité.

La neurotoxicité périphérique chronique et cumulative est considérée comme l'effet indésirable dose-limitant de l'oxaliplatine et peut avoir comme conséquence l'interruption du traitement. La neurotoxicité se manifeste par des symptômes pouvant inclure une neuropathie sensorielle périphérique, une atteinte sensitive, une ataxie sensorielle, et/ou un déficit de coordination fine sensori-motrice. La neuropathie se développe progressivement chez approximativement 10 à 15% des patients après une dose cumulée de 780 à 850 mg/m2, correspondant à approximativement six cycles à la dose de 85 mg/m2 (Gamelin et al., Semin Oncol. 2002 29:21-33). La neuropathie sensorielle périphérique apparaît sous forme de paresthésies et de dysesthésies distales (main, pied, orteil), avec perte de sensation. La neuropathie sensitive, l'ataxie sensorielle, et/ou le déficit de coordination fine sensori-motrice se traduit par des difficultés pour écrire, tenir des objets, *etc.* Le développement de la neuropathie sensitive est corrélé avec la dose cumulée d'oxaliplatine (Wilson et al., J Clin Oncol. 2002 20:1767-74).

Des travaux sur l'animal ont abouti à la mise au point d'un modèle de neurotoxicité induite par infusion unique d'oxaliplatine avec les principaux symptômes rencontrés chez l'homme, en particulier l'hypersensibilité au froid (Ling et al., Toxicology. 2007 234:176-84; Ling et al., Pain. 2007 128:225-34).

Plus généralement, la neurotoxicité est un effet indésirable commun à de nombreux agents anticancéreux. Ainsi, les taxanes et la vincristine induisent une neuropathie chez l'homme (Quastoff and Hartung, J Neurol. 2002 249:9-17; Stillman and Cata, Curr Pain Headache Rep. 2006 18:321-324; Park et al., Curr Med Chem. 2008 15 :3081-3094). US-A-2008/124333 divulgue la combinaison du riluzole et d'un agent anticancéreux, par exemple le carboplatine ou le paclitaxel. WO-A-2006/099739 divulgue l'utilisation de polypeptides ADNF pour le traitement de la neurotoxicité, y compris l'hyperalgésie thermique, induite par des agents anticancéreux. WO-A-2009/028605 divulgue une méthode de criblage pour identifier des composés capables d'atténuer les troubles nerveux périphériques suite à une thérapie anticancéreuse. Lefebvre et al. divulguent dans Soc. Neurosc., 26 (1-2), 2000, 1216, que le prétraitement avec le riluzole, la lamotrigine ou la gabapentine réduit l'hypersensitbilité au froid ainsi que l'hypersensibilité mécanique et le développement de douleurs neuropathiques suite aux lésions neuronales dans un modèle CCI (chronic constriction injury). Il existe donc un besoin en composés capables de traiter ou de prévenir les effets indésirables induits par des agents anticancéreux, en particulier des agents anticancéreux présentant une neurotoxicité.

### DESCRIPTION DE L'INVENTION

Il a été trouvé que le riluzole permet de prévenir l'apparition d'effets indésirables liés à l'administration de l'oxaliplatine.

Plus particulièrement, les inventeurs ont évalué le potentiel du riluzole à réduire les phénomènes d'hypersensibilité au froid. Dans un modèle animal, il a été trouvé que le riluzole est capable de corriger l'hypersensibilité au froid induite par l'oxaliplatine. Cet effet important laisse attendre une action bénéfique du riluzole sur tous les symptômes sensoriels, en particulier de la neurotoxicité, observés lors de l'administration d'oxaliplatine chez l'homme.

De ce fait, un premier aspect de l'invention concerne un composé de formule (I) : 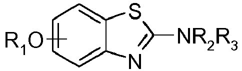 dans laquelle :
- R1 est choisi parmi un atome d'hydrogène, un groupe alkyle en C1-C6 et un groupe halogénoalkyle en C1-C6 ;
- R2, R3, identiques ou différents, sont choisis parmi un atome d'hydrogène, un groupe alkyle en C1-C6,
ainsi que les sels pharmaceutiquement acceptables de ce composé, pour une utilisation dans le traitement ou la prévention d'un effet indésirable, cet effet indésirable étant la neurotoxicité, induit par un agent anticancéreux.

La présente description divulgue aussi l'utilisation d'un tel composé de formule (I), pour la fabrication d'un médicament pour le traitement ou la prévention d'un effet indésirable, en particulier de la neurotoxicité, induit par un agent anticancéreux.

R1 peut par exemple être un groupe halogénoalkyle, de préférence *per-*halogénoalkyle. Préférentiellement, R1 est un groupe perfluoroalkyle, tel que le trifluorométhyle.

R2 et/ou R3 peuvent par exemple représenter un atome d'hydrogène.

Dans un mode de réalisation particulier, le composé de formule (I) est caractérisé en ce qu'il s'agit du composé de formule (II): 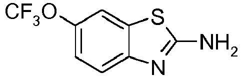 ou un sel pharmaceutiquement acceptable de ce composé de formule (II).

Selon la présente invention, les radicaux Alkyle représentent des radicaux hydrocarbonés saturés, en chaîne droite ou ramifiée, de 1 à 6 atomes de carbone, de préférence de 1 à 4 atomes de carbone. On peut notamment citer, lorsqu'ils sont linéaires, les radicaux méthyle, éthyle, propyle, butyle, pentyle, et hexyle. On peut notamment citer, lorsqu'ils sont ramifiés ou substitués par un ou plusieurs radicaux alkyle, les radicaux isopropyle, tert-butyl, 2-méthylbutyle, 2-méthylpentyle, et 1-méthylpentyle.

Les radicaux halogénoalkyles selon la présente invention désignent des radicaux alkyles tels que définis ci-dessus dans lesquels un ou plusieurs atomes d'hydrogène sont substitués par un atome d'halogène (fluor, chlore, brome, iode), notamment par un atome de chlore ou de fluor. Les radicaux halogénoalkyles peuvent être des radicaux *per*-halogénoalkyle, i.e. des radicaux halogénoalkyles dans lesquels tous les atomes d'hydrogène sont substitués par un atome d'halogène, notamment des radicaux *per*-fluoroalkyles de formule CₙF₂ₙ₊₁ où n représente un nombre entier compris entre 1 et 6. Comme exemple de groupe *per*-fluoroalkyle, on peut citer notamment le trifluorométhyle.

Les effets indésirables, en particulier la neurotoxicité, peuvent être traitée à tout stade. Par « *traitement* », on entend traitement à titre curatif (visant à soulager, freiner ou stopper les effets indésirables). Par « *prévention* », on entend traitement à titre prophylactique (visant à réduire le risque d'apparition des effets indésirables).

L'expression « *sel pharmaceutiquement acceptable »* fait référence aux sels d'addition acide relativement non toxiques, inorganiques et organiques, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide on trouve les sels bromhydrate, chlorhydrate, sulfate, bisulfate, phosphate, nitrate, acétate, oxalate, valerate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-b-hydroxynaphtoates, acide gentisique, iséthionates, di-p-toluoyltartrates, méthanesulfonates, éthanesulfonates, benzènesulfonates, p-toluènesulfonates, cyclohexyl sulfamates et quinateslaurylsulfonate, et analogues. (Voir par exemple S.M. Berge et al. « Pharmaceutical Salts » J. Pharm. Sci, 66 :p.1-19 (1977)). Dans un mode de réalisation préféré, ledit composé de formule (I) est le riluzole (lequel est représenté par la formule (II) ci-dessus). Par « riluzole », on entend le 2-amino-6-trifluoro-méthoxy-benzothiazole, ainsi que ses sels pharmaceutiquement acceptables. Le riluzole peut par exemple être préparé selon le procédé décrit dans le brevet EP 0 050 551. L'utilisation du riluzole pour le traitement de pathologies neurodégénératives est par exemple décrit dans le brevet US 6,872,739 et la demande de brevet EP 0 558 861.

Dans un mode de réalisation préféré de l'invention, l'agent anticancéreux est un sel de platine. Les sels de platine sont couramment utilisés lors du traitement de cancers. Cependant, les sels de platine induisent de nombreux effets indésirables, incluant la neurotoxicité, la myélosuppression, la néphrotoxicité, l'ototoxicité, des nausées et des vomissements.

Dans le cadre de la présente invention, l'effet indésirable correspond à la neurotoxicité, laquelle se manifeste notamment par une hypersensibilité au froid et/ou des troubles musculaires. Toutefois, il existe d'autres effets indésirables, tels que la myélosuppression, la néphrotoxicité, l'ototoxicité, des nausées ou des vomissements. L'homme du métier pourra se reporter à S. Wolf et al., European Journal of Cancer 2008, 44 : 1507-1515.

Dans un mode de réalisation, l'effet indésirable est l'hypersensibilité au froid.

Les sels de platine utilisés lors de traitement de cancers incluent notamment l'oxaliplatine (commercialisé sous le nom d'Eloxatine©), le cisplatine (CDDP, commercialisé sous le nom de Cisplatyl©) et le carboplatine (CDCP, commercialisé sous le nom de Paraplatine©). L'oxaliplatine, le cisplatine et le carboplatine sont donc des sels de platine préférés selon l'invention. De manière particulièrement préférée, le sel de platine est l'oxaliplatine.

L'oxaliplatine est notamment utilisé lors du traitement de cancers du colon, de cancers colorectaux, de cancers de l'ovaire et de cancers de l'estomac. Son association avec le 5-fluoro-uracile et l'acide folinique s'appelle le protocole FOLFOX. Par rapport aux autres sels de platine, ce médicament est peu hématotoxique et n'a pas de toxicité rénale. En revanche, il présente une forte neurotoxicité. Il est responsable de neuropathies sensitives particulières, exagérées par le froid, et dont la sévérité augmente avec la dose cumulée.

Le cisplatine est la base de nombreux protocoles de chimiothérapie, et est notamment utilisé pour le traitement des cancers du testicule, de cancers de la prostate, de cancers du col de l'utérus, de cancers de l'ovaire, de cancers de l'endomètre, de cancers du poumon, de cancers de la vessie, de cancers des voies aérodigestives, de sarcomes et de neuroblastomes. Ses toxicités principales sont rénales (néphrotoxicité tubulaire) et neurologiques (neurotoxicité).

Découvert en 1975, le carboplatine possède deux groupes carboxylates à la place des deux atomes de chlore pour le cisplatine. Il est utilisé pour le traitement de cancers du testicule, de cancers de l'ovaire, de cancers des voies aérodigestives et de cancers du poumon. Par rapport au cisplatine, il a une moindre toxicité rénale et neurologique. En revanche, il existe un risque plus marqué de saignements.

L'invention porte également sur un composé de formule (I), en particulier le riluzole, pour une utilisation dans le traitement ou la prévention d'un effet indésirable, cet effet indésirable étant la neurotoxicité, induit par un agent anticancéreux autre qu'un sel de platine. En effet, la plupart des agents anticancéreux présentent des effets indésirables, et notamment une neurotoxicité. Les inventeurs ayant démontrés qu'un composé de formule (I) convient au traitement et/ou à la prévention de la neurotoxicité induite par l'oxaliplatine, il est possible d'en déduire que le composé de formule (I) convient également au traitement et/ou à la prévention de la neurotoxicité induite par d'autres agents anticancéreux.

Dans un mode de réalisation, l'effet indésirable est l'hypersensibilité au froid.

Par « *agent anticancéreux* », on entend ici tout principe actif utilisé pour le traitement du cancer. Les agents anticancéreux incluent notamment les agents alkylants, les antimétabolites, les agents intercalants, les poisons du fuseau mitotique, les antibiotiques antitumoraux et les modificateurs de la structure tertiaire de l'ADN tel que les inhibiteurs de topoisomérases. L'agent anticancéreux selon l'invention est un agent anticancéreux qui induit une neurotoxicité en tant qu'effet indésirable. C'est notamment le cas des poisons du fuseau mitotique, comme les vinca-alcaloïdes et les taxanes.

Par « *vinca-alcaloïde* » ou « *alcaloïde de la pervenche* », on entend ici un alcaloïde pouvant être extrait de la pervenche (*Vinca rosea* et *Catharanthus* roseus, par exemple) ou un alcaloïde dérivé de celui-ci. Les vinca-alcaloïdes incluent la vincristine, la vinblastine, la vindésine et la vinorelbine.

Par « *taxane* », on entend ici un terpène pouvant être extrait d'un if (*Taxus*) ou un terpène dérivé de celui-ci. Les taxanes incluent le paclitaxel (commercialisé sous le nom de Taxol©) et le docétaxel (commercialisé sous le nom de Taxotère©).

C'est aussi le cas des sels de platine, comme mentionné *supra.*

Dans le cadre de la présente invention, le composé de formule (I), plus particulièrement le riluzole, est préférentiellement utilisé en combinaison avec l'agent-anticancéreux dont il prévient les effets indésirables. L'administration du composé de formule (I) et de l'agent anticancéreux peut être simultanée ou séquentielle.

L'agent anticancéreux peut être présent au sein de la même composition pharmaceutique que le composé de formule (I). L'invention concerne donc une composition pharmaceutique contenant un véhicule pharmaceutiquement acceptable et, en tant que principes actifs, un composé de formule (I), plus particulièrement le riluzole, ainsi qu'un agent anticancéreux, plus particulièrement un sel de platine. Suivant un mode de réalisation, la composition pharmaceutique comprend le riluzole et un sel de platine. Suivant un autre mode de réalisation, la composition pharmaceutique comprend le riluzole et l'oxaliplatine.

Alternativement, l'agent anticancéreux peut être présent dans une composition pharmaceutique séparée de celle contenant le composé de formule (I). Un aspect de la divulgation concerne donc une combinaison (kit) contenant (i) une première composition pharmaceutique contenant un véhicule pharmaceutiquement acceptable et un composé de formule (I) ; et (ii) une seconde composition pharmaceutique contenant un véhicule pharmaceutiquement acceptable et un agent anticancéreux. Un kit selon la divulgation peut éventuellement comprendre des instructions concernant le mode d'administration de la première et de la seconde composition pharmaceutique pour le traitement et la prévention d'un cancer. L'utilisation (administration) de la première et de la seconde composition pharmaceutique d'une telle combinaison (kit) peut être simultanée ou séquentielle. Suivant un mode de réalisation, la première composition pharmaceutique comprend le riluzole et la deuxième composition pharmaceutique comprend un sel de platine. Suivant un autre mode de réalisation, la première composition pharmaceutique comprend le riluzole et la deuxième composition pharmaceutique comprend l'oxaliplatine.

Par « *véhicule pharmaceutiquement acceptable* », on entend tout solvant, milieu de dispersion, agent retardant l'absorption, *etc.,* qui ne produit pas de réaction secondaire, par exemple allergique, chez l'humain ou l'animal. Les véhicules pharmaceutiquement acceptables sont bien connus de l'homme de l'art, et incluent ceux décrits dans « Remington's Pharmaceutical Sciences » (Mack Publishing Company, Easton, USA, 1985). Le véhicule pharmaceutiquement acceptable est notamment choisi en fonction de la voie d'administration, qui peut par exemple être orale, sous-linguale, nasale, buccale, rectale, parentérale (i.e. intraveineuse, sous-cutanée, intradermique ou intramusculaire). La voie d'administration est de préférence orale ou parentérale. La dose dépend de facteurs tels que le principe actif considéré, le mode d'administration, l'indication thérapeutique, l'âge, le poids et l'état du patient.

De manière avantageuse, les compositions pharmaceutiques selon la divulgation et les combinaisons (kits) selon la divulgation sont utilisés pour le traitement ou la prévention d'un cancer. Le cancer peut être choisi parmi un cancer du testicule, un cancer de la thyroïde, un cancer de l'ovaire, un cancer du col de l'utérus et/ou de l'endomètre, un cancer du sein, un cancer de l'oesophage, un cancer de la vessie, un cancer de la sphère oto-rhino-laryngologée (ORL), un cancer du poumon, un cancer de l'estomac, un cancer de la prostate, un cancer du poumon, un sarcome, un neuroblastome, un cancer colorectal, un cancer rectal et un cancer du colon.

Dans un mode de réalisation particulièrement préféré, les compositions pharmaceutiques selon la divulgation et les combinaisons (kits) selon la divulgation contiennent de l'oxaliplatine en tant que sel de platine, du riluzole en tant que composé de formule (I), et sont utilisés pour le traitement ou la prévention d'un cancer colorectal. Dans de telles compositions pharmaceutiques et combinaisons (kits) selon la divulgation, le riluzole permet de traiter ou de prévenir les effets indésirables, en particulier la neurotoxicité induite par l'agent anti-néoplasique qu'est l'oxaliplatine. Un aspect de la divulgation concerne également une méthode de traitement ou de prévention d'un effet indésirable induit par un agent anticancéreux, comprenant l'administration d'une quantité thérapeutiquement efficace d'un composé de formule (I), plus particulièrement du riluzole, à un individu. Cet individu peut être un individu qui a été traité par l'agent anticancéreux, qui est en cours de traitement par l'agent anticancéreux ou qui va être traité par l'agent anticancéreux. Les différentes caractéristiques développées ci-dessus s'appliquent à cette méthode. Ainsi, l'agent anticancéreux peut être un sel de platine, notamment l'oxaliplatine. Ainsi également, l'effet indésirable peut être la neurotoxicité, par exemple l'hypersensibilité au froid. L'individu est de préférence un mammifère, plus particulièrement un humain. La dose thérapeutique efficace peut facilement être déterminée par l'homme du métier. Un aspect de la divulgation concerne également une méthode de traitement ou de prévention d'un cancer, comprenant l'administration d'une quantité thérapeutiquement efficace d'un composé de formule (I), plus particulièrement du riluzole, en combinaison avec un agent anticancéreux, plus particulièrement un sel de platine, notamment l'oxaliplatine, à un individu en ayant besoin. Suivant une caractéristique de cet aspect la méthode est une méthode de traitement ou de prévention d'un cancer, combinée à une méthode de traitement ou de prévention d'un effet indésirable lié à l'agent anticancéreux utilisé. Par combinaison avec un agent anticancéreux, il faut comprendre que l'individu peut être un individu qui a été traité par l'agent anticancéreux, qui est en cours de traitement par l'agent anticancéreux ou qui va être traité par l'agent anticancéreux. L'individu est de préférence un mammifère, plus particulièrement un humain. La dose thérapeutique efficace peut facilement être déterminée par l'homme du métier. Les différentes caractéristiques développées ci-dessus s'appliquent à cette méthode. Ainsi, l'agent anticancéreux peut être un sel de platine, notamment l'oxaliplatine. Ainsi également, l'effet indésirable peut être la neurotoxicité, par exemple l'hypersensibilité au froid.

Enfin, un autre aspect de la divulgation concerne une méthode de criblage pour l'identification d'un composé convenant au traitement ou à la prévention d'un effet indésirable induit par un agent anticancéreux, ladite méthode comprenant les étapes suivantes :
a) administrer un agent anticancéreux à un animal modèle non humain ;
b) administrer un agent anticancéreux et un analogue de synthèse du riluzole à un animal modèle non humain;
c) mesurer un effet indésirable induit par le sel de platine à l'étape (a) et (b) ;
d) comparer l'effet indésirable mesuré en présence et en l'absence dudit analogue de synthèse du riluzole; et, optionnellement,
e) sacrifier les animaux ayant été utilisés lors des étapes (a) et (b)
où la mesure d'un effet indésirable significativement plus faible en présence dudit analogue de synthèse du riluzole, comparativement à celui mesuré en l'absence dudit analogue de synthèse du riluzole, indique que ledit analogue de synthèse du riluzole convient au traitement ou à la prévention d'un effet indésirable induit par un agent anticancéreux.

Par « *analogue de synthèse du riluzole* », on entend un composé chimique dérivé du riluzole. Par dérivé du riluzole, on entend notamment un composé comprenant le squelette benzothiazolique plus ou moins substitué. De tels analogues incluent mais ne sont pas limités aux composés de formule (I).

Ainsi, les composés définis dans EP 0 282 971 sont considérés ici comme des analogues de synthèse, de sorte que l'analogue de synthèse est un composé qui répond à la formule (III) suivante : 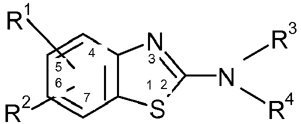 dans laquelle :
R¹ et R², qui peuvent être identiques ou différents l'un de l'autre, représentent un atome d'hydrogène, un radical alkyle linéaire ou ramifié comportant de 1 à 6 atomes de carbone, un radical C₁-C₆-alkyl-C₆-C₁₀-aryle, un radical C₁-C₆-alkényle, phényle, un groupe CF₃, hydroxy, un radical C₁-C₆-alkoxy, C₁-C₆-alkylthio, C₁-C₆-alkylsulfonyle, CF₃O en position 6, un atome d'halogène, un groupe nitro, carboxy, un radical C₁-C₆-alkoxy-carbonyle, un groupe NR⁵R⁶CO, NR⁵R⁶, R⁵CONR⁵, CN, CR⁵R⁶SO₂ ;
dans lesquels :
R⁵ et R⁶, qui peuvent être identiques ou différents l'un de l'autre, représentent un atome d'hydrogène, un radical C₁-C₆-alkyle, ou un radical C₆-C₁₀-aryle ,
R¹ et R² peuvent former ensemble un cycle carbocyclique ou méthylènedioxy ; R³ représente un atome d'hydrogène ;
R⁴ représente un atome d'hydrogène, un radical C₁-C₆-alkyle, un radical C₁-C₆-alkyle substitué par un groupe hétérocyclique ou un groupe hétérocyclique substitué, un groupe méthylcycloalkyle dont la partie cycloalkyle contient jusqu'à 6 atomes de carbone ; un groupe benzyle, phénétyle, phényle, phényle substitué, un radical C₁-C₆-alkyle, propargyle ;
à condition que R¹, R² et R³ doivent représenter des atomes d'hydrogène lorsque R⁴ n'est pas un atome d'hydrogène.

Le composé III est un dérivé sélectionné dans le groupe consistant en :
2-amino-6-méthylbenzothiazole,
2-amino-6-trifluorométhoxybenzothiazole,
2-amino-6-trifluorométhylbenzothiazole,
2-aminobenzothiazole,
2-amino-4-méthylbenzothiazole,
2-am ino-6-éthoxybenzoth iazole,
2-amino-6-nitrobenzothiazole,
2-amino-5-méthoxybenzothiazole,
2-amino-6-méthylsulfonylbenzothiazole,
2-amino-4,6-diméthylbenzothiazole,
éthylaminobenzothiazole,
2-benzylaminobenzothiazole.
2-amino-4-trifluorométhylbenzothiazole,
2-amino-5-trifluorométhylbenzothiazole,
2-amino-6-bromobenzothiazole,
2-am ino-6-chlorobenzoth iazole,
2-am ino-4-chlorobenzoth iazole,
2-amino-6-fluorobenzothiazole,
2-amino-5-méthoxybenzothiazole,
2-amino-4,6-difluorobenzothiazole,
2-amino-6-méthylthiobenzothiazole,
2-amino-naphto[1,2-d]thiazole, et
2-[[2-(1-méthyl-2-pyrrolidinyl)éthyl]amino]-benzothiazole.

L'effet indésirable mesuré dans la cadre de la méthode de criblage selon un aspect de la divulgation peut par exemple correspondre à la neurotoxicité, la myélosuppression, la néphrotoxicité, l'ototoxicité, la nausée ou les vomissements induits par un sel de platine. Dans un mode de réalisation préféré de la divulgation l'effet indésirable est la neurotoxicité. Dans un mode de réalisation préféré de la divulgation l'effet indésirable est l'hypersensibilité au froid.

La neurotoxicité induite par un sel de platine peut par exemple être mesurée comme décrit dans l'exemple 2, en mesurant l'effet du composé de formule (I) sur l'hypersensibilité au froid induite par un sel de platine chez un rat comme animal modèle. Des animaux modèles permettant de mesurer la neurotoxicité induite par un taxane ou par la vincristine sont connus dans l'art, et peuvent également être utilisés (Authier et al., Brain Res. 2000 291 :73-76 ; Authier et al., Neurotherapeutics. 2009 6 :620-629 ; Authier et al., Neurotoxicology. 2003 24:797-805).

Les exemples et figures suivants illustrent l'invention.

### DESCRIPTION DES FIGURES

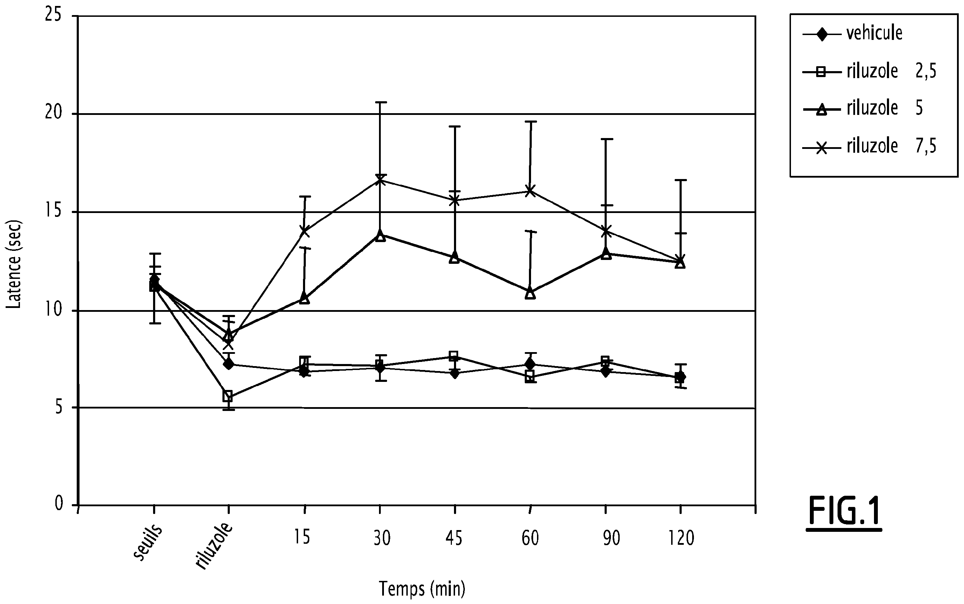

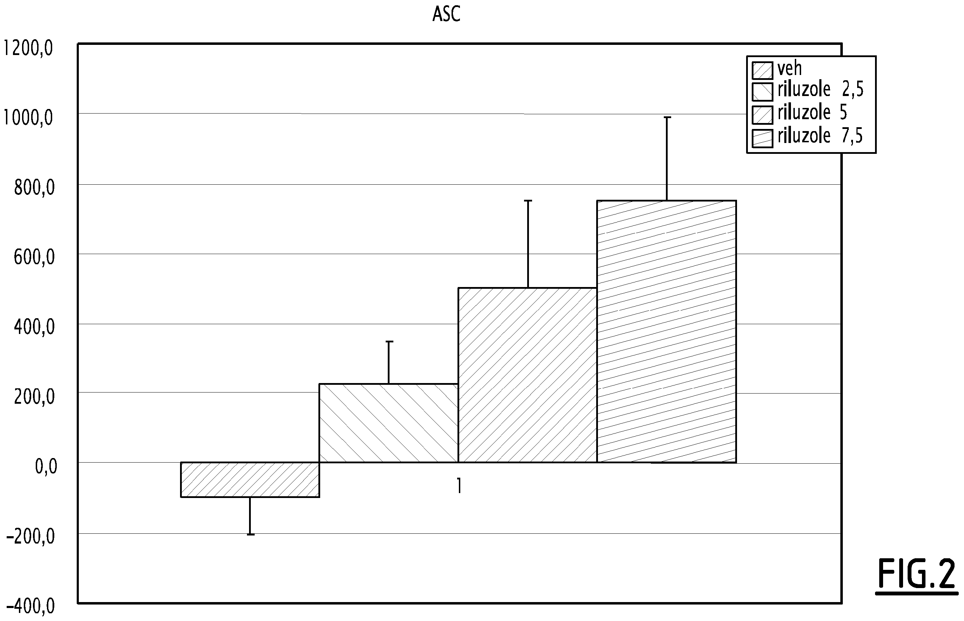

Les figures 1 et 2 montrent que le riluzole est capable de corriger l'hypersensibilité au froid induite par injection d'oxaliplatine.

### EXEMPLES

### EXEMPLE 1 : Protocoles

### 1.1. Animaux

Des rats mâles Sprague-Dawley (Charles River Lab, France), pesant de 175 à 200 grammes, ont été placés en cage avec nourriture et eau *ad libitum,* en environnement thermo régulé à 22°C avec un cycle jo ur/nuit de 12h/12h. Les expérimentations ont été réalisées en aveugle dans une pièce calme par le même expérimentateur en prenant soin de respecter les exigences réglementaires sur l'expérimentation animale.

### 1.2. Molécules

Les molécules suivantes ont été utilisées : riluzole (Sigma Chemical Co., St Louis, MO) et oxaliplatine (Debiopharm, Lausanne, Suisse). Le riluzole a été préparé dans du NaCl (0.9%). L'oxaliplatine a été dilué dans une solution de glucose (5%).

### 1.3. Modèle de neurotoxicité chimio-induite par l'oxaliplatine chez le rat

Chaque rat reçoit par voie intra péritonéale (IP) soit une dose d'oxaliplatine, soit une injection contrôle de glucose 5%. Le test comportemental, l'immersion de la queue du rat dans l'eau à 10°C, est réalisé avant et 72 heures après l'injection de l'oxaliplatine ou du véhicule. Puis le riluzole, aux doses de 2.5, 5 ou 7.5 mg/kg, ou son véhicule, est administré en sous-cutané et les animaux sont à nouveaux testés selon la cinétique suivante : à 15, 30, 45, 60, 90, et 120 minutes après l'injection de riluzole ou de véhicule.

### 1.4. Test d'immersion de la queue du rat dans l'eau à 10°C

Ce test d'immersion à basse température permet de mesurer l'hypersensibilité au froid chez les animaux. La queue des rats est immergée au tiers dans un bain marie thermostaté à 10°C jusqu'au retrait de celle-ci ou jusqu'au « cut-off » fixé à 30 secondes (Necker and Hellon. 1978 Pain. 4:231-242).

### 1.5. Analyses statistiques

Les données expérimentales ont été analysées en utilisant le logiciel Sigma STAT, version 3.0 pour Windows (STAT32 Software Inc., San Diego, CA). La cinétique des délais de réaction à la stimulation thermique a été analysée à l'aide d'une analyse de variance à deux facteurs suivie d'un test de multiple comparaison pour étudier l'évolution dans le temps. Les scores des lots traités au riluzole et des lots témoins ont été comparés par un test t de student. Le seuil de significativité est P<0 ,05.

### EXEMPLE 2 : Effet du riluzole sur la sensibilité au froid chez le rat souffrant de neurotoxicité induite par l'oxaliplatine

Les effets de trois doses de riluzole (2.5, 5 et 7.5 mg/kg) injecté par voie intra péritonéale ont été testés à l'aide du test thermique d'immersion de la queue dans l'eau à 10°C, avant et 72 heures après injection de l'oxaliplatine. L'oxaliplatine diminue le seuil de sensibilité au froid (10°C) (Figure 1). Le rat non traité enlève sa queue au bout de 12 secondes environ, alors que le rat traité par l'oxaliplatine l'enlève au bout de 7 secondes et cet effet persiste pendant tout le temps de l'expérience.

La Figure 1 montre clairement que les délais de réaction du rat traité à l'oxaliplatine sont considérablement augmentés après injection de riluzole dès la dose de 5 mg/kg. Les animaux oxaliplatine traités par le riluzole ont même un délai de réaction au froid supérieur (moins de sensibilité) à celui observé avant injection d'oxaliplatine. L'analyse statistique des données a fait apparaître des différences significatives aux doses de 5 mg/kg (p < 0.05) et 7.5mg/kg (p < 0.001) (Fig. 1, Fig. 2)

### EXEMPLE 3 : Conclusion

Le potentiel du riluzole à réduire les phénomènes d'hypersensibilité au froid a été évalué. Dans un modèle animal, le rat, le riluzole a corrigé l'hypersensibilité au froid induite par l'oxaliplatine. Cet effet important suggère que le riluzole pourrait avoir une action bénéfique sur tous les symptômes sensoriels observés lors de la neurotoxicité associée au traitement de pathologies humaines par l'oxaliplatine.

## Revendications

1. Composé de formule (I) : 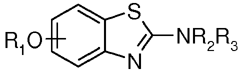 dans laquelle :
- R1 est choisi parmi un atome d'hydrogène, un groupe alkyle en C1-C6, et un halogénoalkyle en C1-C6 ;
- R2, R3, identiques ou différents, sont choisis parmi un atome d'hydrogène et un groupe alkyle en C1-C6 ;
ainsi que les sels pharmaceutiquement acceptables de ce composé, pour une utilisation dans le traitement ou la prévention d'un effet indésirable induit par un agent anticancéreux, ledit effet indésirable étant la neurotoxicité.

2. Composé pour utilisation selon la revendication 1, où l'effet indésirable induit par l'agent anticancéreux est l'hypersensibilité au froid.

3. Composé pour utilisation selon la revendication 1 ou 2, où ledit agent anticancéreux est un sel de platine, un taxane ou un vinca-alcaloïde.

4. Composé pour utilisation selon la revendication 3, où ledit agent anticancéreux est l'oxaliplatine, le cisplatine ou le carboplatine.

5. Composé pour utilisation selon la revendication 4, où ledit agent anticancéreux est l'oxaliplatine.

6. Composé pour utilisation selon la revendication 3, où ledit agent anticancéreux est la vincristine, la vindésine ou la vinorelbine.

7. Composé pour utilisation selon la revendication 3, où ledit agent anticancéreux est le paclitaxel ou le docétaxel.

8. Composté pour utilisation selon l'une quelconque des revendications 1 à 7, où ledit composé de formule (I) est le riluzole.

## Patentansprüche

1. Verbindung der Formel (I): 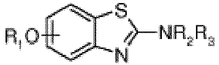 in der:
- R1 ausgewählt ist aus einem Wasserstoffatom, einer C1-C6 Alkylgruppe und einem C1-C6 Halogenalkyl;
- R2, R3, identisch oder unterschiedlich, ausgewählt sind aus einem Wasserstoffatom und einer C1-C6 Alkylgruppe;
sowie pharmazeutisch annehmbare Salze dieser Verbindung für eine Verwendung bei der Behandlung oder der Prävention einer unerwünschten Wirkung durch einen Anti-Krebswirkstoff, wobei die unerwünschte Wirkung die Neurotoxizität ist.

2. Verbindung zur Verwendung nach Anspruch 1, bei der die unerwünschte durch den Anti-Krebs-Wirkstoff induzierte Wirkung die Hypersensibilität gegen Kälte ist.

3. Verbindung zur Verwendung nach Anspruch 1 oder 2, bei der das Anti-Krebs-Mittel ein Salz des Platins, ein Taxan oder ein Vinca-Alkaloid ist.

4. Verbindung zur Verwendung nach Anspruch 3, bei der das Anti-Krebs-Mittel Oxaliplatin, Cisplatin oder Carboplatin ist.

5. Verbindung zur Verwendung nach Anspruch 4, bei der das Anti-Krebs-Mittel Oxaliplatin ist.

6. Verbindung zur Verwendung nach Anspruch 3, bei der das Anti-Krebs-Mittel Vincristin, Vindesin oder Vinorelbin ist.

7. Verbindung zur Verwendung nach Anspruch 3, bei der das Anti-Krebs-Mittel Paclitaxel oder Docetaxel ist.

8. Verbindung zur Verwendung nach einem beliebigen der Ansprüche 1 bis 7, bei der die Verbindung der Formel (I) Riluzol ist.

## Claims

1. Compound of formula (I): 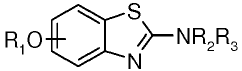 in which:
- R1 is chosen from a hydrogen atom, a C1-C6 alkyl group and a C1-C6 halogenoalkyl group;
- R2, R3, identical or different, are chosen from a hydrogen atom and a C1-C6 alkyl group,
as well as the pharmaceutically acceptable salts of this compound, for use in the treatment or prevention of an adverse effect induced by an anticancer agent, said adverse effect being neurotoxicity.

2. Compound for the use according to claim 1, in which the adverse effect induced by the anticancer agent is hypersensitivity to cold.

3. Compound for the use according to claim 1 or 2, in which said anticancer agent is a platinum salt, a taxane or a vinca alkaloid.

4. Compound for the use according to claim 3, in which said anticancer agent is oxaliplatin, cisplatin or carboplatin.

5. Compound for the use according to claim 4, in which said anticancer agent is oxaliplatin.

6. Compound for the use according to claim 3, in which said anticancer agent is vincristine, vindesine or vinorelbine.

7. Compound for the use according to claim 3, in which said anticancer agent is paclitaxel or docetaxel.

8. Compound for the use according to any one of claims 1 to 7, in which said compound of formula (I) is riluzole.
